# EUROPEAN PATENT APPLICATION

(11) **EP 0 571 057 A1**
(43) Date of publication of application: **24.11.1993**
(21) Application number: 93250137.2
(22) Date of filing: 12.05.1993
(51) Int. Cl.: A61B 17/28, A61B 17/04

(54) **Needle holder for endoscopic surgery**

(30) Priority: 21.05.1992 DE 4216875
(71) Applicant: ETHICON INC., Somerville, New Jersey 08876-0151 (US)
(72) Inventor: Wohlers, Udo, W-2000 Hamburg 65 (DE)
(74) Representative: UEXKÜLL & STOLBERG

(57) **Abstract**

The invention relates to a gripping instrument, in particular a needle holder, for endoscopic surgery. The needle holder consists of a front forceps zone with clamping jaws, an instrument tube (17) in which a longitudinally displaceable pull rod (3) is guided, a rearward handle arrangement which, when actuated, drives an adjustment element (7) connected to the pull rod (3), and a spring element (20) for the limitation of force from the handle arrangement. A force-limiting coupling is formed according to the invention in that the adjustment element (7) comprises at its distal end a sleeve arrangement (6, 8, 12) in which the proximal end zone of the pull rod (3) is guided displaceable in longitudinal direction. A second abutting surface (5) is provided at the distal end of the sleeve arrangement (6, 8, 12) and a first abutting surface (9) is secured at the pull rod (3) in the zone inserted into the sleeve arrangement (6,8,12). Seated between the first (9) and the second abutting surface (5) is a compression spring (20) which is compressed upon loading of the adjustment element (7) as soon as there is resistance to the further movement of the clamping jaws and therefore of the pull rod (3). This prevents an overloading of the clamping jaws and of the whole instrument when the handle arrangement is pressed together.

## Description

The invention relates to a gripping instrument, in particular a needle holder, for laparoscopic operations which consists of a front zone having clamping jaws, an instrument tube with a pull rod guided in axially displaceable manner in same, a rearward handle arrangement which, when actuated, drives an adjustment element connected to the pull rod, and a spring element for the limitation of force from the handle arrangement onto the pull rod.

In modern minimally invasive surgery, the instruments needed for the procedure are introduced into the inside of the body, for example into the abdominal cavity, through cannulae and guided under the control of a likewise introduced endoscope or under X-ray observation and actuated for the individual operation steps. Such operations techniques are used for example in laparoscopic cholecystectomy and appendectomy. Intraabdominal sutures must be provided in these and similar operations. The sutures are created using special surgical needles which are held from outside with a needle holder introduced through a cannula and guided by the operator. Such needle holders are provided with two clamping jaws which are movable towards each other to grasp a needle in the manner of forceps between the clamping jaws. One or both of the clamping jaws are opened and closed by a push- or pull-rod guided in the instrument tube of the needle holder.

The position at which the needle lies between the clamping jaws is not predetermined, i.e. the needle can lie well to the rear, near the axis of rotation of the clamping jaw, so that when they grasp the needle the clamping jaws remain relatively widely opened, or it can lie well forward, near the distal ends of the clamping jaws, so that when they grasp the needle the clamping jaws are largely closed. Efforts must be made to ensure that the force exerted by the clamping jaws is as independent as possible of the extent to which the handle or the clamping jaws are closed.

Another problem with such holders or forceps is that, because the handle is very large in relation to the clamping jaws, it is very difficult for the user to measure the force exerted on the clamping jaws and the clamping jaws are frequently pressed against each other with too great a contact pressure if the operator closes the handle, while the clamping jaws encounter a resistance lying between them. This can easily result in damage to the clamping jaws, to their bearings or in the transmission of force, and the life of the instrument is reduced overall through overloading. It is known per se in this regard to provide force-limiting mechanisms in the transmission from the handle onto the clamping jaws.

With the medical forceps described in DE-OS 40 10 775 with a fixed and a swivellable jaw part, a handle arrangement with a fixed and a swivellable handle part is provided, the movement of the swivellable handle part being transmitted via a push- or pull rod onto the swivellable jaw part. To limit the compressive force at the mouth parts, the swivellable handle part is divided into two parts which are connected to each other by a bending spring element. As the bending spring element is deflected when the jaw parts encounter resistance, the force onto the jaw parts is limited even if the handle is closed to the greatest possible extent.

The object of the invention is to create a gripping instrument, in particular a needle holder, for endoscopic surgery in which the action of force on the clamping jaws is limited regardless of the closure position of the handle arrangement and which can be carried out regardless of the special shape of the handle arrangement.

This object is achieved by a gripping instrument, in particular a needle holder, which is formed according to the features of claim 1 or according to the features of claim 4. Advantageous versions of the invention are listed in the subsidiary claims.

In the embodiment of claim 1 according to the invention the adjustment element, which is driven in longitudinal direction upon closure of the handle arrangement, is provided at its distal end with a sleeve arrangement into which the pull rod is inserted with its proximal end zone and is displaceably guided therein. The sleeve arrangement comprises at its distal end a first abutting surface against which a compression spring lies. The compression spring rests with its opposite end on a second abutting surface which is attached to the pull rod in the zone inserted into the sleeve arrangement. As the pull rod is displaceably guided in the sleeve arrangement, a tensile force exerted by the adjustment element on the sleeve arrangement is transmitted from the first abutting surface via the compression spring onto the second abutting surface and thus the pull rod. If, upon closure of the clamping jaws, there is resistance to the further movement of the pull rod, the compression spring influences the force exerted on the adjustment element if the handle arrangement is closed further. As a result, when the closure of the handle arrangement is complete the displacement of the adjustment element is not fully transmitted onto the pull rod and therefore the force exerted on the clamping jaw secured at the pull rod is limited.

The term sleeve arrangement refers to any formations of two coaxially lying rods in which one rod is displaceable in longitudinal direction in or at the other.

In the embodiment according to claim 4, the distal end of the adjustment element, which is driven essentially in longitudinal direction upon closure of the handle arrangement, is connected to the proximal end of a helical spring, the longitudinal axis of which is aligned essentially in extension of the pull rod. The distal end of the helical spring is connected to the proximal end of the pull rod. If, upon closure of the clamping jaws, there is resistance to the further movement of the pull rod, the helical spring is tensioned upon further closure of the handle arrangement, and the force transmitted onto the pull rod is limited to the force by which the helical spring is tensioned or stressed. It is particular advantageous when the helical spring is pre-tensioned. In this case the length of the helical spring does not change before the force acting upon the helical spring exceeds the given pre-tensioning force.

The invention is explained below, with reference to embodiments, in the drawings which show:
- Figure 1: an overall view of a needle holder;
- Figure 2: the front part of the needle holder with the clamping jaws;
- Figure 3: a first embodiment of the arrangement according to the invention for the transmission of force from the handle arrangement onto the pull rod of the needle holder; and
- Figur 4: an overall view of a needle holder including a second embodiment of the arangement according to the invention for the transmission of force from the handle arrangement onto the pull rod.

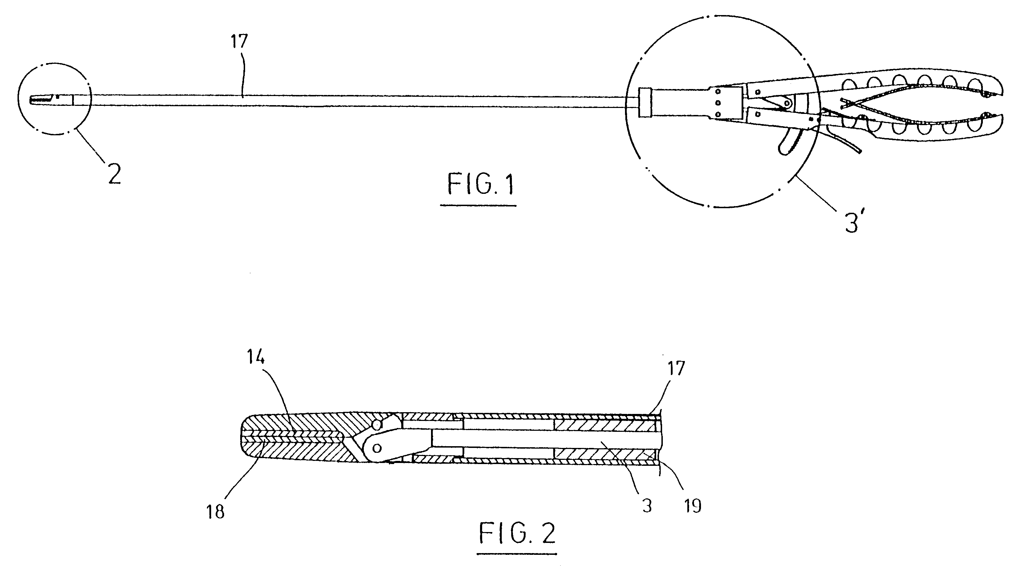

Figure 1 shows a whole needle holder with the clamping jaw zone 2 lying at the distal end, the instrument tube 17 connecting thereto, and the transmission mechanism 3' from the rearward handle arrangement onto the pull rod guided in the instrument tube 17.

In Figure 2, the clamping jaw zone at the distal end is shown magnified. The needle holder comprises a fixed clamping jaw 18 and a swivellable clamping jaw 14 to which the pull rod 3 is hingedly connected. In the instrument tube 17, the pull rod 3 is guided at seal surfaces 19.

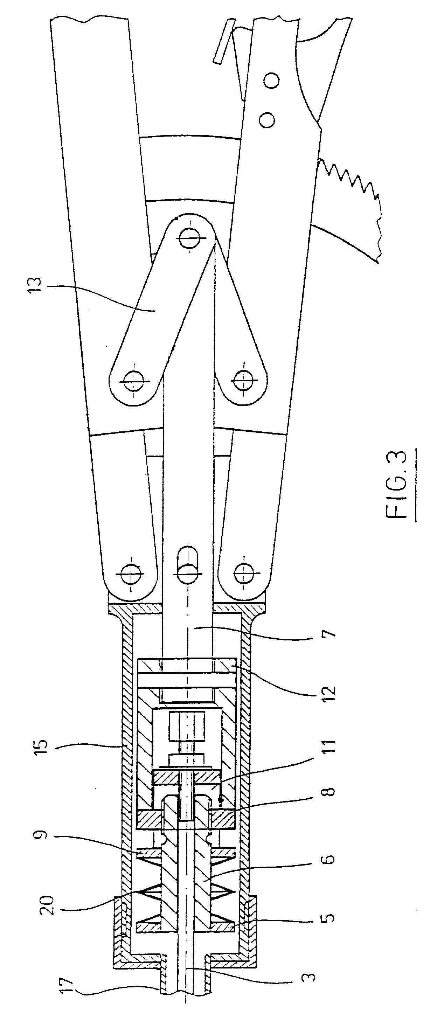

In Figure 3, the arrangement for force transmission is shown magnified in section. Arranged in the instrument tube 17 is the longitudinally displaceable pull rod 3 which is coupled with the movable clamping jaw 14 at the front forceps zone 1 (shown magnified in Figure 2). The forceps-like handle arrangement at the rearward end of the instrument is actuated by closure of the handle parts towards each other, whereby a longitudinal movement, to the right in the representation of Figure 3, is transmitted via the lever 13 onto the adjustment element 7. In Figure 3, the adjustment element 7 is already in the retracted position. The adjustment element 7 is firmly connected to a sleeve arrangement which comprises a bush segment 12, i.e. a cylindrical casing, at which lateral zones are removed on both sides, parallel to the cylinder axis. A disk segment 8, formed to correspond to the cross-section of the bush segment 12, attaches to the bush segment 12 at its distal end. The disk segment 8, at which lateral zones are removed corresponding to the bush segment 12, is secured at a cylindrical bush 6. The cylindrical bush 6 has a central bore.

The pull rod 3 is displaceably guided in the bush 6 of the sleeve arrangement 6, 8, 12. Attached to the pull rod 3 in the zone inside the bush segment 12 is a bracket 11 which embraces the disk segment 8 and carries at its distal end an abutting surface 9 in the form of a disk which encloses the bush 6 but is displaceable against it. Lying opposite in distal direction to the first abutting surface 9 is the second abutting surface 5 which is formed by a disk which is firmly connected to the bush 6 of the sleeve arrangement 6, 8, 12. Between the second abutting surface 5, firmly connected to the adjustment element 7, and the first abutting surface 9, firmly connected to the pull rod 3, lies a compression spring 20. The compression spring 20 is formed by a sequence of cup springs lying one behind the other. By this means a high elasticity constant can be provided on a short section. The elasticity constant and therefore the maximum force exerted on the jaw elements can also be pre-set through the number of cup springs.

The proximal end of the pull rod 3 lies, in the unloaded case of the compression spring 20, against an abutment surface of the bush segment 12 so that, upon opening of the handle arrangement, the pushing movement of the adjustment element 7 is transmitted onto the pull rod 3 and the clamping jaw 14 is opened.

In the embodiment shown in Figure 3 the compression spring 20 is pre-tensioned in a way as to press the first abutting surface 9 away from the second abutting surface 5, up to a stop, as long as the force exerted by the adjustment element 7 does not exceed the pre-tensioning force of the compression spring 20.

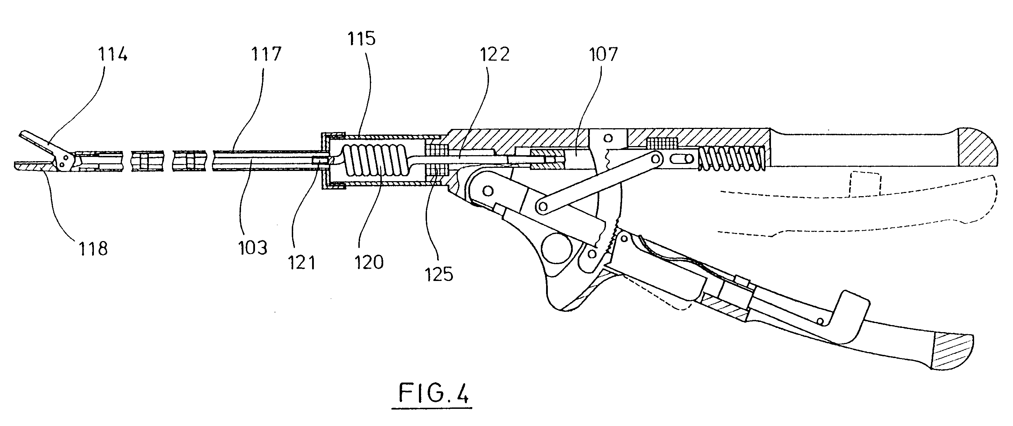

Figure 4 shows another embodiment in which the pull rod and the adjustment element are directly connected to each other via a helical spring, without involving a sleeve arrangement. Parts corresponding to those of the embodiment according to Figures 1 to 3 are identified by reference numerals increased by 100.

The design of the handle arrangement and the transmission of force onto the adjustment element 107 is somewhat different from that displayed in Figures 1 and 3. But also in the embodiment according to Figure 4 the adjustment element 107 is moved to the right, i.e. in proximal direction, upon closure (as indicated in dashed lines) of both handle parts. The pull rod 103 is coupled to the adjustment element 107 via a helical spring 120. The helical spring 120, which is inside a housing 115 connecting the instrument tube 117 with the handle arrangement, has its longitudinal axis aligned in extension of the pull rod 103 and in extension of the adjustment element 107. The distal end zone 121 of the helical spring 120 is formed to a straight section extending along the longitudinal axis and being rigidly connected to the proximal end of pull rod 103. The proximal end zone 122 of helical spring 120 is formed to a straight section as well, which is guided by the central opening of a mounting bush 125 and which is attached to the distal end of adjustment element 107.

In the state displayed in Figure 4 the helical spring 120 is in its resting position. Preferably it is pre-tensioned in this state. When the handle parts are moved towards each other this movement is transmitted by the helical spring 120 onto the pull rod 103, while the helical spring 120 does not elongate, and the clamping jaw 114 swivels onto the fixed clamping jaw 118. If, however, there is resistance to the further movement of the clamping jaws 114 and 118, the helical spring 120 is elongated upon further compression of the handle parts as soon as the pre-tensioning force of the helical spring 120 is exceeded. Thus the force transmitted to pull rod 103 is limited to the force by which the spring is tensioned or stressed, and it cannot assume undesirably high values.

The arrangements according to the invention for the limitation of the force exerted from the handle arrangement onto the pull rod are not limited to the application in needle holders. They are applicable to all kinds of gripping instruments, which also include forceps-like instruments, even those having jaws provided with a cutting edge, and they can also be used in other instruments, in particular in endoscopic instruments, in which the force transmitted from a handle arrangement onto an actuating element is to be limited to a given maximum value.

## Claims

1. Gripping instrument, in particular needle holder, for endoscopic surgery which consists of a front clamping jaw zone (14, 18), an instrument tube (17) with a pull rod (3) guided in axially displaceable manner therein, a rearward handle arrangement which, when actuated, drives an adjustment element (7) connected to the pull rod (3), and a spring element (20) for the limitation of force from the handle arrangement onto the pull rod, **characterized in that**
the adjustment element (7) comprises at the distal end a sleeve arrangement (6, 8, 12) in which the proximal end zone of the pull rod (3) is housed and is displaceably guided therein in longitudinal direction,
a second abutting surface (5) is provided at the distal end of the sleeve arrangement (6, 8, 12) and a first abutting surface (9) at the pull rod (3) in the zone inserted into the sleeve arrangement, and
a compression spring (20) attaches coaxial to the pull rod (3) between the first (9) and second abutting surface (5).

2. Gripping instrument, in particular needle holder, according to claim 1, **characterized in that** the sleeve arrangement (6, 8, 12) is formed by a bush (6), a disk segment (8) attached to its proximal end and a bush segment (12) attached to the disk segment (8), the second abutting surface (5) being formed by a disk secured at the distal end of the bush (6), and in that there is secured at the pull rod (3) in the zone lying in the bush segment (12) a bracket (11) which embraces the disk segment (8) and carries at its distal end the first abutting surface (9) in the form of a disk coaxially enclosing the bush (6).

3. Gripping instrument, in particular needle holder, according to one of the previous claims, **characterized in that** the compression spring (20) is formed by several cup springs lying one behind the other.

4. Gripping instrument, in particular needle holder, for endoscopic surgery which consists of a front clamping jaw zone (114, 118), an instrument tube (117) with a pull rod (103) guided in axially displaceable manner therein, a rearward handle arrangement which, when actuated, drives an adjustment element (107) connected to the pull rod (103), and a spring element (120) for the limitation of force from the handle arrangement onto the pull rod, **characterized in that** the distal end of the adjustment element (107) is connected to the proximal end (122) of a helical spring (120), the longitudinal axis of which is aligned essentially in extension of the pull rod (103), and that the distal end (121) of the helical spring (120) is connected to the proximal end of the pull rod (103).

5. Gripping instrument, in particular needle holder, according to claim 4, **characterized in that** the helical spring (120) is pre-tensioned.
